(19) Europäisches Patentamt
European Patent Office
Office européen des brevets

(11) Publication number: **0 164 389**
**B1**

(12) # EUROPEAN PATENT SPECIFICATION

(45) Date of publication of patent specification: **01.04.87**

(51) Int. Cl.$^4$: **C 07 C 101/08,**
C 07 C 101/18, C 07 C 99/00,
C 07 C 103/30, C 07 C 102/00

(21) Application number: **85900162.0**

(22) Date of filing: **07.12.84**

(86) International application number:
**PCT/HU84/00060**

(87) International publication number:
**WO 85/02609 20.06.85 Gazette 85/14**

(54) **PREPARATION OF PHENYL ALANINES BY HYDROGENATION OF PHENYL SERINES.**

(30) Priority: **08.12.83 HU 419583**
**22.12.83 HU 419583**

(43) Date of publication of application:
**18.12.85 Bulletin 85/51**

(45) Publication of the grant of the patent:
**01.04.87 Bulletin 87/14**

(84) Designated Contracting States:
**AT BE CH DE FR GB LI LU NL**

(56) References cited:
**DE-A-2 741 081**
**DE-A-2 845 216**
**DE-A-3 019 069**
**FR-A-2 277 575**

(73) Proprietor: **ALKALOIDA VEGYéSZETI GYáR**
**Postfach 1**
**H-4440 Tiszavasvári (HU)**

(72) Inventor: **CSORVASSY, István**
**26, Kazinczy u.**
**H-4080 Hajdunánás (HU)**
Inventor: **KORODI, Ferenc**
**9, Elmunkás u.**
**H-4440 Tiszavasvári (HU)**
Inventor: **SALAMON; Zoltan**
**17-19, Kabay J. u.**
**H-4440 Tiszavasv-ri (HU)**
Inventor: **SANDOR, Jvzsef**
**8, Jvkai u.**
**H-4450 Tiszalßk (HU)**

(74) Representative: **Skailes, Humphrey John et al**
**Frank B. Dehn & Co. Imperial House 15-19**
**Kingsway**
**London WC2B 6UZ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 164 389 B1

**Description**

The present invention invention relates to a large scale process for the preparation of 3-phenyl alanine derivatives of the general formula

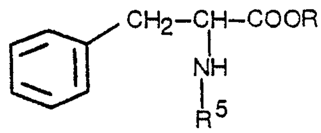

I

In the general formulae as referred to hereinafter the symbols are defined as follows:

R stands for hydrogen or $C_{1-4}$ alkyl

$R^5$ stands for hydrogen or $-CO-R^4$ wherein

$R^4$ stands for hydrogen $C_{1-4}$ alkyl, aralkyl or aryl

Background Art

Phenyl alanine is a known essential amino acid. Recently its importance has increased since it is the active ingredient of the sweetening agent known as "aspartam"[methyl-L-aspartyl-L-phenyl-alaninate]. L-Phenyl alanine is prepared by fermentation in the industry. This process is rather expensive as at the end of the fermentation the product has to be isolated from a big volume of water containing a great amount of inorganic ions, and the purification of the water is rather complicated [EU Pat. Appln. 85 959].

Several classical amino acid synthesis methods are known for the preparation of phenyl alanine: such as the Strecker synthesis using the expensive phenyl acetaldehyde [Bull. Chem. Soc. Jap. 46. [1978] 1865—66] or the Erlenmayer azlactone synthesis in several steps from N-acyl glycerine with an average total yield etc. [J. Org. chem. *37.* No. 18 [1972] 2616].

Disclosure of the invention

The present invention relates to the preparation of phenyl alanine derivatives of the general formula I by reacting a phenyl serine derivative of the general formula

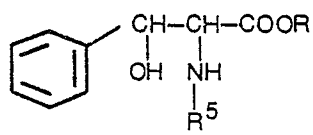

II

or a salt thereof formed with inorganic or organic acid with hydrogen in the presence of a catalyst and optionally N-acylating said product.

When using gaseous hydrogen palladium metal may be used as a catalyst, preferably in the form of 10% palladium on charcoal in an amount of 10 to 50% by weight related to the weight of the starting material.

The reaction is performed in a protic solvent in the presence of $C_{1-4}$ alcohol, water or a $C_{1-4}$ straight chain carboxylic acid. A mixture of said solvents may be used as well. As a preferred embodiment glacial acetic acid may be used as a solvent.

One of the possible embodiments of our process can be conducted by introducing hydrogen gas in the presence of 1 to 5 moles of mineral acid relative to the phenyl serine derivative.

Hydrogenation may be conducted at a pressure of 1 to 10 atm., preferably at 3—5 atm. The preferred reaction temperature range is from 20 to 120°C, preferably 40—70°C.

As mineral acids sulfuric acid or hydrochloric acid can preferably be used.

As another preferred embodiment of the process according to the invention the required hydrogen is formed in situ from a substance which releases hydrogen under the conditions of the reaction on the surface of the catalyst in the reaction mixture. As such substances hydrazine, formic acid, cyclohexane or cyclohexadiene are preferred.

As protic solvents particularly carboxylic acids may be used. If in the end product the carboxylic or amino group containing a substituent different from hydrogen then as starting material the corresponding N-substituted compound of the general formula II can be employed. Particularly if esters are prepared phenyl serine is used as starting material which upon hydrogenation results in phenyl alanine ester.

If N-acyl derivatives are to be prepared the acylation can be performed both before and after hydrogenation. Acylation may be carried out by methods known per se, e.g. with carboxylic acid anhydride. [J. Biol. Chem. 98 [1932] page 295].

If the formyl derivatives are prepared then the reaction can preferably be prepared by using formic acid hydrogenating agent and at the same time as acylating agent.

In order to prepare N-formyl phenylalanine of the formula

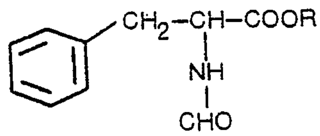

III

falling within the scope of the compounds of the general formula I formic acid is particularly preferred as hydrogen releasing agent. Phenyl serine of the formula

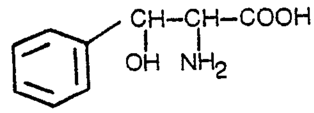

IV

is then used as starting material and by reacting same with formic acid, hydrogenation and the formylation of the amino group can be carried out in one step. For this reaction 1—40%, preferably 10—20% by weight of 10% palladium on charcoal catalyst can be used calculated on the weight of the starting material. The reaction can be performed with 2 to 20 moles formic acid calculated on phenyl serine, preferably in the presence of glacial acetic acid. As a solvent formic acid can be used in the presence of water or without it.

Phenyl alanine and derivatives thereof can be prepared under large scale conditions economically and in good quality by using the process of the invention. Industrial synthesis can be conducted from economically available glycine prepared by large scale synthesis. Phenyl serine derivatives of the general formula II which serve as starting materials for the hydrogenation according to the invention are known and can be prepared from glycine.

According to a preferred embodiment of the invention glycine and benzaldehyde are reacted under alkaline conditions in a two-phase solvent system. One of the two phases consist of water immiscible organic solvent. The other layer comprises a water miscible organic solvent and/or water. The reaction takes place in the presence of a phase transfer catalyst.

As phase transfer catalysts 0.01—1.0 mole equivalent quaternary ammonium salt relative to glycine can be used. Reaction is carried out at 15—100°C, preferably at 25—60°C. The phenyl serine alkali salt thus prepared can be further used in another reaction [e.g. dehydroxylation], without isolation. As water immiscible solvents aromatic hydrocarbons, such as benzene or toluene may be used. As water miscible organic solvents $C_{1-4}$ alcohol or acetone may be used.

Phenyl alanine prepared according to the invention may be in optically active form or racemic form. As starting material erithro or threo D-, L- or DL-phenyl serine may be employed. If the DL-form is used racemic phenyl alanine is formed according to the process of the invention. The product is then resolved by method known per se [J. Prakt. Chem. *9.*[1959]104.].

Further details of the invention can be found in the following Examples which serve merely for illustration and not for limitation.

Best mode of carrying out the Invention

Example 1

To a mixture of 200C ml of glacial acetic acid, 135 ml of 96% sulfuric acid and 20 ml water, 50 g of 10% palladium on charcoal catalyst are added and the mixture is vigorously stirred at atmospheric pressure under hydrogen. To the thus prehydrated mixture 100 g [0.552 mole] of DL-threo-3-phenyl-serine monohydrate are added and the reaction mixture is further stirred at 20°C. When the theoretical amount of hydrogen [13.0 l] is consumed the catalyst is removed by filtration, washed with 2 × 100 ml of hot glacial acetic acid, whereafter 308.3 g of anhydrous sodium acetate are added to the combined filtrates, the precipitated salt is filtered, washed with 2 × 100 ml of hot glacial acetic acid and the glacial acid layer is evaporated in vacuo at 30—40°C. The obtained crystalline residue is dissolved in 800 ml methanol and the pH is adjusted to pH=4.5 by adding concentrated aqueous ammonia solution, the mixture is cooled and the precipitated product is filtered. Yield: 68.6 g of DL-3-phenyl-alanine. [75.2%]

Example 2

In a mixture of 200 ml of glacial acetic acid, 80 ml of 96% sulfuric acid and 200 ml of water 10 g of 10% palladium on charcoal are added and the mixture is vigorously stirred under hydrogen atmosphere at 5—60°C for 20 minutes. 100 g of DL-phenyl serine are then added and the mixture is hydrogenated under similar conditions for 12 hours. The catalyst is filtered, washed twice with 50 ml of hot glacial acetic acid and the mixture is then boiled. 180.0 g of anhydrous calcium chloride are then added to the hot solution in small portions. When the addition is completed the solution is hot filtered and worked up according to Example 1. 75.5 g of DL-phenyl alanine are obtained. Yield: 82.7%.

Example 3

2.20 g of DL-phenyl serine monohydrate are dissolved in 20 ml of glacial acetic acid whereafter 1.0 g of palladium on charcoal is added. 12.0 ml of formic acid are added dropwise, the purity of which is 98%. The mixture is maintained at 95°C for 4 hours, filtered and the filtrate is evaporated and the residue is

recrystallized from acetone. Yield: 2.07 g of DL-N-formyl-alanine. Yield: 86.7%. Purity: 98% M.p. 168—170°C.

Example 4

2.1 g of L-phenyl serine ethyl ester are dissolved in 15 ml of glacial acetic acid, 0.80 g of palladium on charcoal is added and 20 ml of hydrazine are added dropwise. The mixture is kept at 100°C for 6 hours, filtered and evaporated in vacuo. The product is purified by column chromatography. 1.3 g of L-phenyl-alanine-ethyl ester is obtained. Yield: 65.4%, purity: 95%. Similarly cyclohexane or cyclohexediene may be used instead of hydrazine hydrate.

Example 5

5.0 g of N-acetyl-phenyl serine are dissolved in 60 ml of glacial acetic acid and 2.0 g of palladium on charcoal and 30 ml formic acid of 98% purity are added and the reaction mixture is kept at 80°C for 3 hours. The mixture is filtered, the filtrate is evaporated and the residue is recrystallized from a mixture of ethanol and water. 4.44 g of DL-N-acetyl phenyl alanine are obtained. Yield: 92.0%. Purity: 96.0%, m.p.: 142—143°C.

Example 6

6.0 g of L-N-formyl-phenyl serine are dissolved in 80 ml of glacial acetic acid, 1.5 g of palladium on charcoal are added as well as 35 ml of formic acid of purity of 98%. The mixture is kept at 100°C for 4.5 hours and worked up according to Example 5. It is crystallized from formic acid. 5.59 g of L-N-formylphenyl alanine are obtained. Yield 96%. Purity: 95%. M.p.: 169—170°C. $[\alpha]_D^{20}=+73°$ [c=l, ethanol].

Example 7

30 g of 10% palladium on charcoal catalyst are admixed with 100 ml of water whereafter 200.0 g of phenyl serine monohydrate are added in 1400 ml of 85% formic acid in nitrogen atmosphere. The mixture is boiled for 4 hours, filtered while hot and the catalyst is washed with hot formic acid and the filtrate is evaporated. To the obtained white crystalline substance 300 ml of 20% hydrochloric acid are added and the mixture is heated under reflux for 1 hour, cooled, stirred at 0—5°C for 2 hours and the precipitated crystalline substance is filtered, washed with some 20% hydrochloric acid and dried. Yield: 92.9%.

187.4 g of DL-3-phenyl alanine hydrochloride are obtained. M.p.: 239—240°C.

Example 8

30 g of 10% palladium on charcoal catalyst are heated in a mixture of 540 ml of glacial acetic acid and 90 ml of 85% formic acid for 20 minutes whereafter a solution of 200 g phenyl serine monohydrate in 540 ml of 85% formic acid and 360 ml of glacial acetic acid are added dropwise at 106—107°C within 2 hours. The mixture is then boiled up for further 2.5 hours and the mixture is worked as given above. Yield: 95.4%. 192.3 g of DL-3-phenyl-alanine hydrochloride.

Example 9

40 ml of glacial acetic acid and 6 ml of formic acid are admixed and in the mixture 2.3 g of 10% palladium on charcoal catalyst are suspended and the mixture is heated under stirring to reflux temperature [105—110°C]. At this temperature a solution of 22.6 g of ammonium salt of N-formyl-phenyl serine in 100 ml of glacial acetic acid and 16 ml of formic acid is added dropwise within 1 hour and it is heated to boiling for 1 hour. The catalyst is filtered while hot, the mixture is washed with some hot glacial acetic acid, the filtrate is evaporated from water bath to 30 to 32 g in vacuo. To the obtained syrup 30 ml of 20% hydrochloric acid are added, the solution is boiled for 1 hour and evaporated from water bath in vacuo. The residue [28—32 g] is taken up in 200 ml 90% methanol and the pH is adjusted to 6—7 with c.c aqueous ammonia. The solution is allowed to stand overnight in a refrigerator, the precipitated product is filtered, washed with methanol and dried to constant weight. Yield: 15.5 g. [84.6%], of DL-3-phenyl alanine. Purity: 90%. The filtered catalyst can be used in the next reaction without further treatment according to all Examples.

Example 10

1.5 g of 10% palladium on charcoal catalyst are suspended in 5 ml water and a solution of 9.80 g of L-phenyl-serine methyl ester and 3.5 g of ammonium formate in 75 ml of 85% formic acid is added. The solution is boiled for 3 hours and filtered, the filtrate is evaporated in vacuo 17.4 g of syrup like residue are allowed to stand at room temperature for 48 hours in 200 ml methanol in hydrochloric acid. The mixture is evaporated and the residue is triturated with ether, the obtained crystalline substance is filtered and dried. 8.62 g [80.1%] of L-phenyl alanine methyl ester hydrochloride are obtained. M.P.: 158—160°C. $[\alpha]_D^{25°}=+32.2°$ [c=2, ethanol].

Example 11

In 1500 ml of water 80.0 g [2.0 mole] of sodium hydroxide and 75.0 g [1.0 mole] of glycine are dissolved at 15°C 212.0 g [2.0 mole] of benzaldehyde and 10.0 g of triethyl benzyl ammonium hydroxide are added in 200 ml of benzene, the mixture is stirred for 2 hours at 50°C, cooled, whereafter 150 ml of glacial acetic acid are added and the two layers are separated. From the benzene layer 105.0 g of benzaldehyde are recovered. The aqueous layer is evaporated in vacuo to 590.0 g. To the residue 1000.0 ml of 100% formic acid are added and the obtained homogeneous solution is added to 20.0 g 10% palladium on charcoal suspended in 1000 ml glacial acetic acid. The mixture is boiled for 3 hours, the catalyst is filtered off and the filtrate is evaporated in vacuo, to the residue 300 ml of concentrated hydrochloric acid are added, the mixture is boiled for 1 hour, then cooled and stirred for 2 hours at 0—5°C. The precipitated DL-3-phenyl alanine hydrochloride is filtered, washed with some 1:1 hydrochloric acid, dried. Yield: 244.8 g containing 160.6 g of hydrochloride of phenyl alanine [79.7%] and 84.2 g sodium chloride. The obtained product is stirred in 1000 ml absolute methanol at room temperature [25°C], and the undissolved sodium chloride is removed by filtration, the filtrate is adjusted to pH=6.5 with concentrated aqueous ammonia and the mixture is allowed to stand overnight at 0—5°C. The precipitated crystals are filtered, washed with some 80% methanol, dried. Yield: 122.1 g [74%] of DL-3-phenyl alanine.

Example 12

1.5 g 10% palladium on charcoal catalyst is covered with 5 ml water and a solution of 10.4 g N-acetyl-threo-L-phenyl-serine and 3.5 g of ammonium formiate in 70 ml of formic acid is added. The mixture is boiled for 3 hours, the catalyst is filtered and washed with 5 ml c.c. hydrochloric acid and cooled. The precipitated N-acetyl-3-phenyl-L-alanine is filtered and washed with water. Yield: 8.1 g 78%, m.p.: 169—171°C $[\alpha]_D=+44.8$ [c=2, ethanol].

14.3 g of N-benzoxl-threo-L-phenyl serine $[\alpha]_D=-60.8°$ [c=2, ethanol], 3.5 g ammonium formiate, 35 ml of glacial acetic acid and 32 ml 85% formic acid solution are added to a solution of 3 g 10% palladium on charcoal in 10 ml glacial acetic acid and the mixture is boiled for 5 hours. The catalyst is filtered off, the mixture is evaporated, dissolved in a mixture of 200 ml methanol and 800 ml of water while hot, a mixture of 10 ml methanol and 10 ml of conc. hydrochloric acid is added and the precipitated N-benzoyl-L-phenyl alanine is filtered. Yield: 5.5 g 41%, m.p.: 134—136°C. $\alpha_D=-15.8°$ [c=2, ethanol] $\alpha_D=17.5°$ [c=4, 0.25 m potassium hydroxide solution].

Preparation of the Starting Materials

Example 1

Preparation of DL-threo-3-phenyl serine 12.0 g [0.30 mole] of sodium hydroxide are dissolved in 50 ml water and 10.0 g [0.13 mole] of glycine are added. To the reaction mixture 25 ml of benzene and 1.2 g [0.005 mole] of triethyl benzyl ammonium chloride are added and the mixture is heated to 50°C 28.35 g [0.26 mole] of benzaldehyde are added and the mixture is kept at 50°C for 2 hours, whereafter it is cooled, acidified, filtered and washed with aqueous ethanol. After drying in vacuo at 100°C 21.60 g of DL-threo-3-phenyl serine are obtained. After drying the product on air at 80°C 23.8 g of DL-threo-3-phenyl serine monohydrate are obtained.

Example 2

Acetylation of DL-threo-3-phenyl serine 10.8 g of phenyl serine are dissolved in 11.2 g hydroxide and 80 ml water. At 0°C 16.8 ml [0.18 mole] of acetic acid anhydride are added. After the addition the mixture is stirred for 1 hour at 0°C then for 1 hour at room temperature whereafter 22.8 ml of 36% by weight hydrochloric acid are added at 0°C. The precipitated product is filtered and washed with some water. 11.2 g of DL-threo-N-acetyl-3-phenyl serine are obtained. M.p.: 150—152°C.

Example 3

Formylation of DL-threo-3-phenyl serine 9.0 g [0.05 mole] of DL-threo-3-phenyl serine and 8 ml of formamide are stirred for 2 hours at 100°C. The reaction mixture is cooled and the product is precipitated with 22 ml of acetone, filtered and washed with acetone. 9.5 g [83.7%] of the ammonium salt of DL-threo-2-formylamindo-3-phenyl serine are obtained. M.p.: 169—170°C.

Analytical method for analysing phenyl serine, phenyl alanine and derivatives
Equipment: high pressure liquid chromatograph of type Hewlett-Packard 10843
Column: Hewlett-Packard RP—8 [5u, 200×4.6 mm]
Eluant: A: 0.004M $H_3PO_4$: 0.016 M $KH_2PO_4$, 20% $CH_3OH$
B: $CH_3OH$
Flow rate: 1 ml/ min
Injected volume: 10.0 µl
Detection: UV detector, 215 mm
Quantitative determination: 1 mg/ml [methanol-water] external standard
retention time: min.
1. Eluant: % B 0.0
phenyl serine: 3.70
phenyl alanine: 5.0
2. Eluant: % B 20.0
N-formyl phenyl serine 4.0
N-formyl phenyl alanine 6.0
N-acetyl phenyl serine 4.40
N-acetyl phenyl alanine 7.60
phenyl serine methyl ester 3.70
phenyl alanine methyl ester 4.80

**Claims**

1. Process for the preparation of phenyl alanine derivatives of the general formula

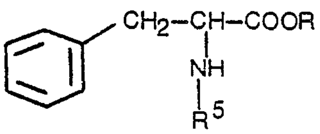
I

wherein
R stands for hydrogen or $C_{1-4}$ alkyl
$R^5$ stands for hydrogen or $-CO-R^4$ wherein
$R^4$ stands for hydrogen, $C_{1-4}$ alkyl, aryl or aralkyl which comprises reacting a phenyl serine derivative of the general formula

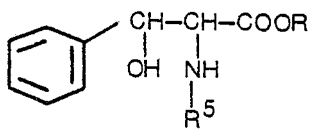
II

or a salt thereof formed with an inorganic or organic acid — wherein
R and $R^5$ are as defined above with hydrogen in the presence of a catalyst and optionally N-acylating said product.

2. Process for the preparation of phenyl alanine, which comprises reacting 3-phenyl serine with hydrogen in the presence of a catalyst and a protic solvent and 1—5 moles of mineral acid relative to 3-phenyl serine.

3. Process as claimed in claim 1, which comprises using metal palladium as catalyst, preferably in the form of 10—50% by weight of 10% palladium on charcoal relative to the weight of the starting material.

4. Process as claimed in claims 1—2, which comprises conducting the reaction by introducing hydrogen gas at 1—10 atm and 20—120°C in the presence of 1—5 moles of mineral acid and a protic solvent.

5. Process as claimed in claims 1—3, which comprises carrying out the reaction in the presence of a protic solvent, such as $C_{1-4}$ alcohol, $C_{1-4}$ straight chain carboxylic acid, water or a mixture thereof, preferably in glacial acetic acid.

6. Process as claimed in claim 1 to 2, which comprises forming the required hydrogen in situ from a substance releasing hydrogen in the reaction mixture on the surface of the catalyst.

7. Process as claimed in claim 5, which comprises using hydrazine, cyclohexane or cyclohexadiene as a hydrogen releasing agent.

8. Process as claimed in claim 1 to 2 and 5, which comprises using formic acid as hydrogen releasing substance.

9. Process as claimed in claims 1, 2, 5 and 7, for the preparation of N-formyl-phenyl-alanine of the general formula

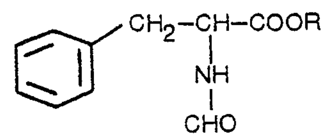

III

which comprises reacting phenyl serine of the formula

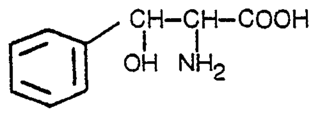

IV

with 2—20 moles formic acid related to phenyl serine in the presence of 40% by weight of 10% palladium on charcoal relative to the weight of phenyl serine.

10. Process as claimed in claim 1, which comprises reacting phenyl serine or a salt thereof with at least 3 moles of formic acid relative to phenyl serine in the presence of palladium on charcoal catalyst and after removing the catalyst reacting the obtained N-formyl-phenyl-alanine with water and mineral acid by method known per se in order to produce a salt of phenyl alanine formed with mineral acid.

**Patentansprüche**

1. Verfahren zur Herstellung von Phenylalanin-Derivaten der allgemeinen Formel:

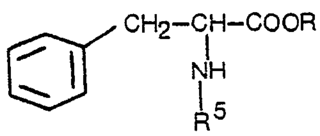

I

worin

R Wasserstoff oder $C_{1-4}$-Alkyl bedeutet,

$R^5$ Wasserstoff oder $-CO-R^4$ darstellt, wobei

$R^4$ Wasserstoff, $C_{1-4}$-Alkyl, Aryl oder Aralkyl bedeutet, gekennzeichnet durch Umsetzung eines Phenylserin-Derivates der allgemeinen Formel

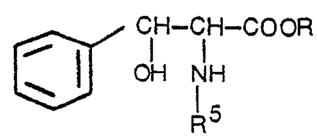

II

oder eines Salzes einer anorganischen oder organischen Säure desselben, wobei R und $R^5$ die vorstehend angegebenen Bedeutungen haben, mit Wasserstoff in Gegenwart eines Katalysators und — sofern dies gewünscht wird — N-Acylierung des Produktes.

2. Verfahren zur Herstellung von Phenylalanin, gekennzeichnet durch Umsetzen von 3-Phenylserin mit Wasserstoff in Gegenwart eines Katalysators und eines protischen Lösungsmittels und 1 bis 5 Mol einer anorganischen Säure, bezogen auf 3-Phenylserin.

3. Verfahren nach Anspruch 1, dadurch gekennzeichnet, dass metallisches Palladium als Katalysator, vorzugsweise in Form von 10 bis 50 Gew.-% 10%igem Palladium auf Kohle, bezogen auf das Gewicht des Ausgangsmaterials, verwendet wird.

4. Verfahren nach den Ansprüchen 1 bis 3, dadurch gekennzeichnet, dass die Reaktion durch Einführung von Wasserstoffgas bei 1 bis 10 Atmosphären und 20 bis 120°C in Gegenwart von 1 bis 5 Mol anorganischer Säure und einem protischen Lösungsmittel erfolgt.

5. Verfahren nach Ansprüchen 1 bis 3, dadurch gekennzeichnet, dass die Reaktion in Gegenwart eines protischen Lösungsmittels, wie einem $C_{1-4}$-Alkohol, einer $C_{1-4}$-geradkettigen Carbonsäure, Wasser oder einem Gemisch derselben, vorzugsweise in Eisessig, durchgeführt wird.

6. Verfahren nach den Ansprüchen 1 bis 3, dadurch gekennzeichnet, dass der erforderliche Wasserstoff in situ aus einer wasserstoffabgebenden Substanz im Reaktionsgemisch auf der Oberfläche des Katalysators gebildet wird.

7. Verfahren nach Anspruch 5, dadurch gekennzeichnet, dass Hydrazin, Cyclohexan oder Cyclohexadien als wasserstofffreisetzendes Agens verwendet wird.

8. Verfahren nach den Ansprüchen 1 bis 2 und 5, dadurch gekennzeichnet, dass Ameisensäure als wasserstofffreisetzenden Substanz verwendet wird.

9. Verfahren nach den Ansprüchen 1, 2, 5 und 7 zur Herstellung von N-Formyl-phenyl-alanin der allgemeinen Formel

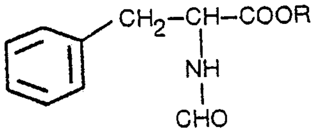 III

gekennzeichnet durch Umsetzung von Phenylserin der Formel

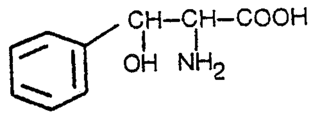 IV

mit 2 bis 20 Mol Ameisensäure, bezogen auf Phenylserin, in Gegenwart von 40 Gew.-% 10%igem Palladium auf Kohle, bezogen auf das Gewicht von Phenylserin.

10. Verfahren nach Anspruch 1, gekennzeichnet durch Umsetzung von Phenylserin oder einem Salz desselben mit mindestens 3 Mol Ameisensäure, bezogen auf Phenylserin, in Gegenwart von Palladiumauf-Kohle-Katalysator und — nach Entfernung des Katalysators — Umsetzung des erhaltenen N-Formyl-phenyl-alanins mit Wasser und einer anorganischen Säure nach einer an sich bekannten Methode, um ein Salz aus Phenylalanin und der anorganischen Säure zu erhalten.

**Revendications**

1. Procédé de préparation de dérivés de phényl-alanine de formule générale

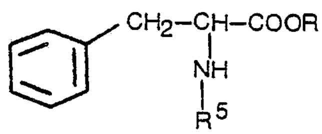 I

où

R représente un hydrogène ou un alcoyle en $C_1$ à $C_4$

$R^5$ représente un hydrogène ou $-CO-R^4$ où

$R^4$ représente un hydrogène, un alcoyle en $C_1$ à $C_4$, un aryle ou un aralcoyle, dans lequel on fait réagir un dérivé de phényl-sérine de formule générale

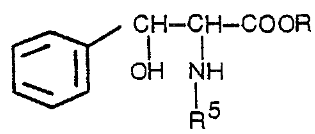 II

ou un se ses sels formés avec un acide inorganique ou organique — où

R et $R^5$ sont tels que définis ci-dessus — avec de l'hydrogène en présence d'un catalyseur et éventuellement on N-acyle ledit produit.

2. Procédé de préparation de phényl-alanine, dans lequel on fait réagir de la 3-phenyl-sérine avec de l'hydrogène en présence d'un catalyseur et d'un solvant protique et de 1—5 moles d'un acide minéral apparenté à la 3-phénylsérine.

3. Procédé selon la revendication 1, dans lequel on utilise du palladium métal comme catalyseur, de préférence sous forme de 10—50% en poids de palladium à 10% sur charbon de bois par rapport au poids du produit de départ.

4. Procédé selon les revendication 1—2, dans lequel on conduit la réaction en introduisant de l'hydrogène gazeux à 1—10 atm. et 20—120°C en présence de 1—5 moles d'acide minéral et d'un solvant protique.

5. Procédé selon des revendications 1—3 dans lequel on conduit la réaction en présence d'un solvant protique, comme un alcool en $C_1$ à $C_4$, un acide carboxylique, à chaîne droite en $C_1$ à $C_4$, d'eau ou d'un de leurs mélanges, de preference dans l'acide acétique glacial.

6. Procédé selon les revendication 1 à 2 dans lequel on forme l'hydrogène requis in situ à partir d'une substance libérant de l'hydrogène dans le milieu réactionnel à la surface du catalyseur.

7. Procédé selon la revendication 5, dans lequel on utilise de l'hydrazine, du cyclohexène ou du cyclohexadiène comme agent libérant de l'hydrogène.

8. Procédé selon les revendications 1 à 2 et 5, dans lequel on utilise de l'acide formique comme substance libérant de l'hydrogène.

9. Procédé selon les revendications 1, 2, 5 et 7, pour la préparation de N-formyl-phényl-alanine de formule générale

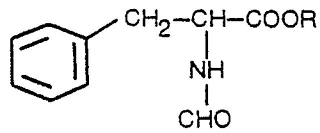

III

dans lequel on fait réagir de la phényl-sérine de formule

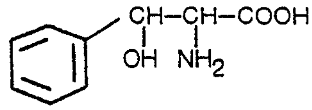

IV

avec 2—20 moles d'acide formique par rapport à la phényl-sérine en présence de 40% en poids de palladium à 10% sur charbon de bois par rapport au poids de la phényl-sérine.

10. Procédé selon la revendication 1, dans lequel on fait réagir de la phényl-sérine ou un de ses sels avec au moins 3 moles d'acide formique par rapport à la phényl-sérine en présence de catalyseur à base de palladium sur charbun de bois et après avoir enlevé le catalyseur on fait réagir la N-formyl-phényl-alanine obtenue avec de l'eau et un acide minéral par un procédé connu pour produire un sel de phényl-alanine forme' avec l'acide minéral.